# EUROPEAN PATENT SPECIFICATION

(11) **EP 4 360 677 B1**
(45) Date of publication and mention of the grant of the patent: **09.07.2025**
(21) Application number: 22306631.7
(22) Date of filing: 28.10.2022
(51) Int. Cl.: A61M 5/32, A61M 5/31

(54) **A SAFETY DEVICE AND A DRUG DELIVERY SYSTEM MOUNTED ON SAID SAFETY DEVICE**
SICHERHEITSVORRICHTUNG UND AUF DIESER SICHERHEITSVORRICHTUNG MONTIERTES ARZNEIMITTELABGABESYSTEM
DISPOSITIF DE SÉCURITÉ ET SYSTÈME D'ADMINISTRATION DE MÉDICAMENT MONTÉ SUR LEDIT DISPOSITIF DE SÉCURITÉ

(43) Date of publication of application: 01.05.2024
(73) Proprietor: Becton, Dickinson and Company, Franklin Lakes, NJ 07417-1880 (US)
(72) Inventor: MILLS, Freddy, 38000 Grenoble (FR); CHARVIN, Matthieu, 38000 Grenoble (FR); CHANSAVANG, Albert, 38000 Grenoble (FR); ALBENGE, Olivier, 38130 Echirolles (FR); BESSON, Nicolas, 38650 Treffort (FR)
(74) Representative: Germain Maureau

(56) References cited:
- FR-A1- 2 778 571
- US-A1- 2003 181 871
- US-A1- 2014 039 406
- US-B2- 7 241 277
- US-B2- 8 298 193

## Description

The present invention relates to a safety device for mounting onto a drug delivery device such as a prefilled or pre-fillable syringe in order to protect a user from needle stick injuries after injection of a medical product. The invention also relates to a drug delivery device including this safety device.

### Background

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction of injection, with respect to the safety device or drug delivery device of the invention and the "proximal direction" is to be understood as meaning the opposite direction to said direction of injection, that is to say the direction towards the user's hand.

Drug delivery devices, such as pre-fillable or prefilled syringes, usually comprise a hollow body or barrel forming a container for a medical product. This body comprises a distal end in the form of a longitudinal tip defining an axial passageway through which the medical product is expelled from the container. The distal end is equipped with a needle for injection of the medical product into an injection site.

It is of great importance that the patients and users are protected from any risk of needle stick injuries, particularly between the moment that injection is finished and the discarding of the drug delivery device.

In order to minimize the risks of needle stick injuries, drug delivery devices may be equipped with a safety device that protects the needle after injection. Safety devices usually comprise a tubular body for receiving the syringe barrel, and a needle cover, in the form of a protective sleeve, that slides relative to the tubular body. The needle cover has a retracted position in which the needle cover is substantially contained inside the tubular body to allow a user to carry out an injection, and an extended position in which the needle cover moves distally from the retracted position to cover the needle once the injection is completed.

Although known injection systems are generally satisfactory, they do not always meet all of the user's expectations and they can include a number of components. Therefore, they can be expensive to manufacture and thus this can limit the widespread use of safety devises at the expense of the security of medical users and patients. Such prior art safety devices are e.g. known from US 2014/039406 A1, which shows the combination of features of the preamble of claim 1. Other prior art safety devices for needle hub applications are known from e.g. US 2003/181871 A1.

They also tend to include components made of various materials which can be complex and expensive to recycle

### Summary

A need exists for an improved safety that provides a user with more convenient safety device that is also cost effective and easy to manufacture.

The invention is defined in claim 1. It is directed to a safety device for mounting onto a drug delivery device having a barrel including with a flange at its proximal end and an injection needle at its distal end, and a piston rod including a piston flange, the safety device including:
a tubular body extending along a longitudinal axis (A), the tubular body being configured to receive the barrel,
a head arrangement configured to abut against the flange and to lock into the flange,
at least two actuation arms connecting the tubular body and the head arrangement, said actuation arms having a proximal link connected to the head arrangement by a proximal hinge and a distal link connected to the tubular body by a distal hinge, the proximal link and the distal link being connected by an intermediate hinge, the actuation arms being movable between a collapsed position in which the tubular body is maintained in a position proximally close to the flange uncovering the needle and an extended position in which the tubular body is maintained in a position distally distant from the head arrangement shielding the needle,
a locking means for releasably maintaining the actuation arms in said collapsed position and a locking means for maintaining the actuation arms in said extended position.

At least one the hinge cab be a living hinge.

The safety device is formed in a single shot injection molding process.

The safety device has a relaxed state in which the arms returns from being in the collapsed position or from being in the extended position.

The proximal link and the distal link form an angle between 75° and 105° in the relaxed position.

The proximal links can include at least one locking hooks configured and sized to enter into resilient snap fit engagement with at least one wings protruding from the annular head to maintain the actuation arm in the extended position.

The head arrangement includes an annular head comprising a locking member which includes a radially oriented wall and two wings extending laterally from the wall.

The annular head includes a longitudinal tongue which extends distally from the locking member and is configured and sized to engage into a channel defined by two guiding walls provided on the tubular body.

The intermediate hinge inwardly overtakes the plan defined by the proximal hinge and distal hinge when the actuation arm is in its extended position.

The annular head can includes a proximal ring and a distal ring jointed by a shoulder whereon the flange abuts.

The distal link can include two orthogonal finger plates.

The annular head comprises at least one locking tab configured to retain the flange.

The tubular body comprises a leg extending in cantilever and having a catch at its free end, the leg being configured to deflect radially outward to allow the catch to slide over the annular head during mounting and to deflect radially inward after mounting completion so that the catch axially retains the tubular body relative to annular head.

The safety device can includes at least one recess positioned at the junction between the shoulder and the distal ring.

The head arrangement can include a crown having a proximal plate configured and sized to lock onto the flange and a distal plate mounted on the tubular body, the proximal plate and the distal plate being connected by at least two actuation arms.

The proximal plate can include two symmetrical ribs, each rib having two locking teeth configured to retain the flange.

The distal link includes at least one looking hook configured and sized to engage into at least one opening provided in the proximal link when the arm is in a collapsed position.

The safety device can includes two legs having a catch, extending in cantilever from the distal plate, configured and sized to engage into a window provided in the proximal plate and to lock into the rib.

The safety device can includes at least one finger tab positioned at the junction of the distal link and the proximal link.

In a second aspect, as defined in claim 20, the invention is directed to a drug delivery device having a barrel including with a flange at its proximal end and an injection needle at its distal end, and a piston rod including a piston flange fitted with a safety device as previously described, wherein the piston flange includes a ramped surface distally convergent configured to outwardly deflect the leg.

### Brief description of the drawings

With reference to the appended drawings, below follows a more detailed description of aspects and embodiments of the invention cited as examples.

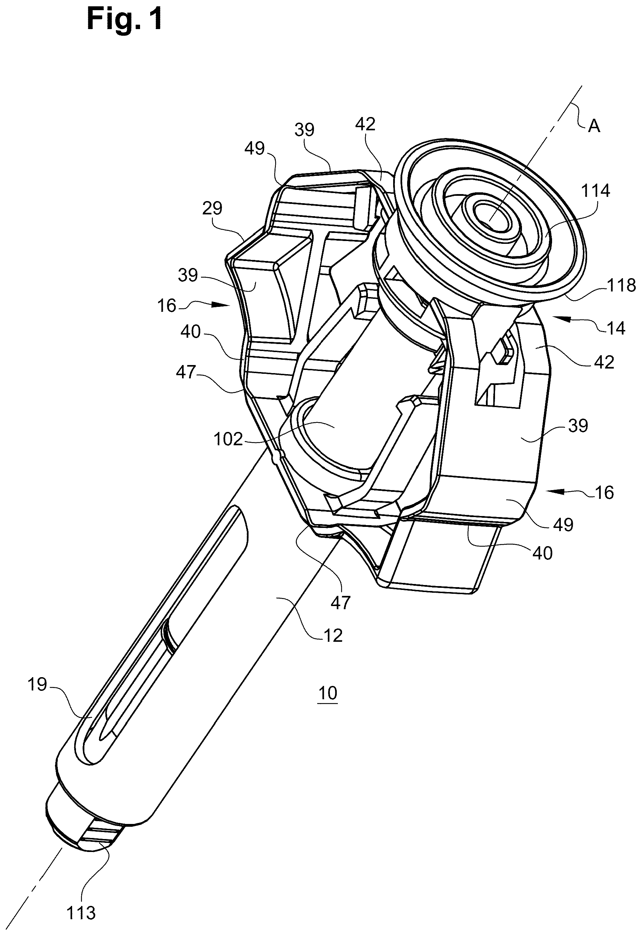

Fig.1 is a perspective view of a safety device according to one embodiment of the invention mounted on a drug delivery device;

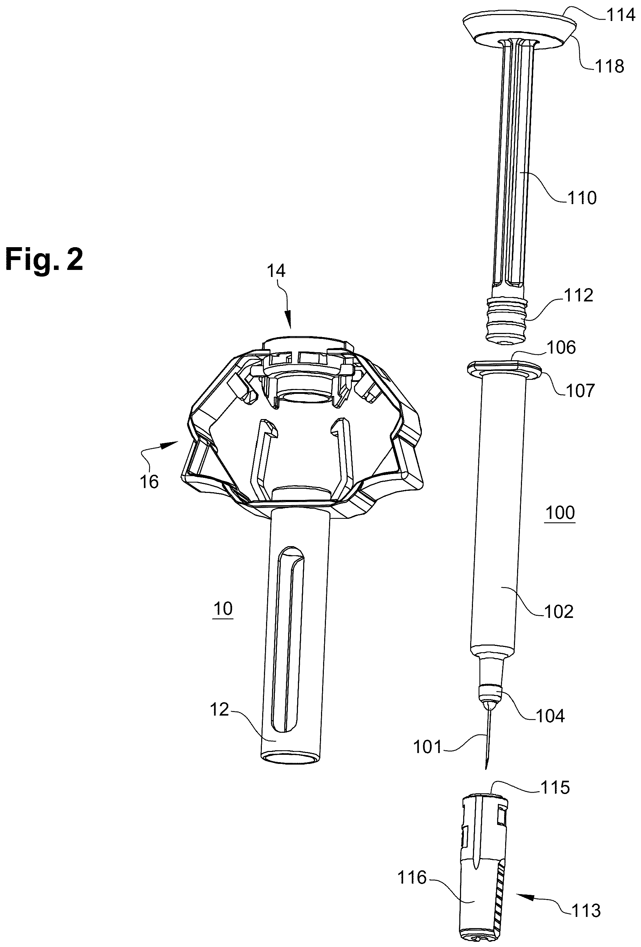

Fig.2 is an exploded view of the safety device and of the drug delivery device of Fig.1;
Fig.3 and 4 are perspective views of the safety device of Fig.1;
Fig.5 is a perspective view of a part of the safety device at a large scale;
Fig.6 and 7 are perspective views of the safety device in a collapsed state;
Fig.8 shows a part of a safety device mounted on a drug delivery device in a pre-injection position;
Fig.9 shows a safety device mounted on a drug delivery device in a pre-injection position;
Fig.10 illustrates an exemplary use of a safety device mounted on a drug delivery device;
Fig.11 illustrates a safety device mounted on a drug delivery during injection;
Fig.12 shows a safety device mounted on a drug delivery device moving into an extended position;
Fig.13, Fig.14 and Fig.15 are perspective views according to different angles of a safety device mounted on a drug delivery device in an extended state;
Fig.16, Fig.17, Fig.18 and Fig 19 show other embodiments of a safety device;
Fig.20 and Fig.21 show another embodiment of a safety device mounted on a drug delivery device;
Figs.22 to 24 show the safety device of Figs 20 and 21 in extended and collapsed positions.

### Description of the invention

The different features of the embodiments can be used in combination with and used with other embodiments as long as the combined parts are not inconsistent with or interfere with the operation of the device and assembly. This invention is not limited in its application to the details of construction and the arrangement of components set forth in the following description or illustrated in the drawings. The embodiments herein are capable of being modified, practiced or carried out in various ways. The phraseology and terminology used herein is for the purpose of description and should not be regarded as limiting. The use of "including," "comprising," or "having" and variations thereof herein is to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. Further, terms such as distal, proximal, up, down, bottom, and top are relative, and are to aid illustration, but are not limiting. The embodiments are not intended to be mutually exclusive so that the features of one embodiment can be combined with other embodiments as long as they do not contradict each other. Terms of degree, such as "substantially", "about" and "approximately" are understood by those skilled in the art to refer to reasonable ranges around and including the given value and ranges outside the given value, for example, general tolerances associated with manufacturing, assembly, and use of the embodiments. The term "substantially" when referring to a structure or characteristic includes the characteristic that is mostly or entirely present in the structure.

With reference to the Figures, a safety device 10 is configured to be mounted onto a drug delivery device 100 to protect an injection needle 101 of the drug delivery device 100 after an injection has been carried out.

The drug delivery device 100 may be a prefilled or prefillable syringe. The drug delivery device 100 includes a tubular barrel 102 defining a reservoir for containing a medical product such as a drug. The tubular barrel 102 may be made of a plastic or of a glass material. This barrel 102 has a distal shoulder 103 provided with a distal tip 104 longitudinally protruding from said shoulder 103 along a longitudinal axis A. The distal tip 104 defines an axial passageway in fluid communication with the reservoir and is equipped with the injection needle 101 for injecting the medical product in an injection site. The barrel 102 further includes an opposite opened proximal end 106 provided with a flange 107. The opened proximal end 106 receives a plunger rod 110 for pushing a stopper 112 located inside the barrel 102 to expel the medical product from the reservoir to the injection site via the distal tip 104 and the injection needle 101.

The drug delivery device 100 may be fitted with a tip cap 113 for protecting and sealing the injection needle 101 before injection. The tip cap 113 may be comprised of a soft inner element 115 and a rigid outer element 116.

The piston rod 110 is fitted with a plunger flange 114 at its proximal end. In some embodiments, the plunger flange 114 can include a ramped surface 118. The ramped surface 118 is conically convergent toward the distal direction.

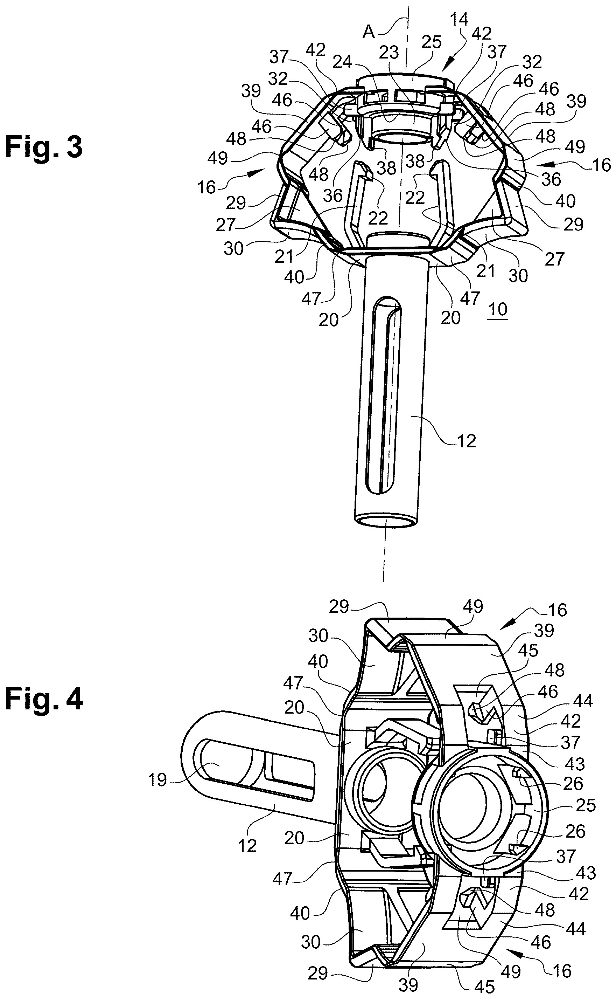

Turning to Fig. 3 and Fig.4, the safety device 10 includes a tubular body 12, which extends along the longitudinal axis A and is configured and sized to engage on the barrel 102 and a head arrangement which can include an annular head 14 configured and sized to engage on the barrel 102 and to lock on the flange 107.

In some embodiment, two symmetrical articulated actuation arms 16 connect the tubular body 12 and the annular head 14. Other embodiments may include three or four actuation arms 16.

The tubular body 12 is a tubular element, which has an inner diameter sized and configured so that the tubular body 12 can axially slide relative to the barrel 102. In practice, the tubular body 12 inner diameter slightly exceeds the external diameter of the barrel 102. In some embodiments, the tubular body 12 can be provided with one or more windows 19 so that any marking or labelling applied on the barrel 102 and so that the drug contained in the barrel 102 can be visible to a user prior to injection. In the illustrated embodiments, the tubular body 12 is provided with two windows 19 spaced apart by 180°.

In some embodiments, the tubular body 12 can comprise two tabs 20 positioned at its proximal end. The tabs 20 extends radially from the outer surface of the tubular body 12 and the tabs 20 can be oriented substantially perpendicular to the longitudinal axis A.

The tubular body 12 further comprises two legs 21. The legs 21 extend in cantilever from each tab 20 in the proximal direction. In some embodiments, the legs 21 can have an arched shape with an inward facing concavity. The cantilevered attachment of the legs 21 onto the tabs 20 combined with its arched shape provide the legs 21 with elastic resilience in a radial direction relative to the tubular body 12. At the end of the legs 21, is a catch 22, which is internally oriented. In some embodiments, the catch 22 has a ramped end.

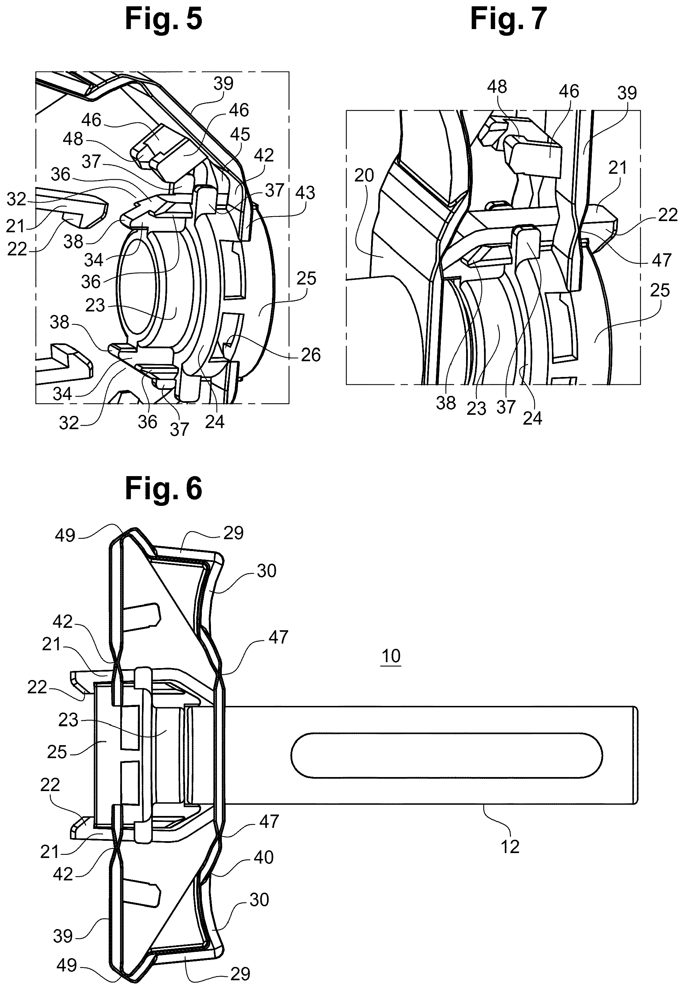

Now turning to Fig.5, the annular head 14 comprises a distal ring 23 attached by a shoulder 24 to a proximal ring 25. The distal ring 23 is coaxial with the tubular body 12 and has an inner diameter, which substantially equates the inner diameter of the tubular body 12.

As can be seen on Fig 4, the proximal ring 25 can be provided with one or more locking tabs 26, which radially protrude from the inner surface of said proximal ring 25. In the illustrated embodiments, the proximal ring 25 includes four locking tabs 26 spaced apart by 90°.

In some embodiments, the annular head 14 further comprises two projecting locking member 32. Each projecting locking member 32, which extends from the shoulder 24 and the distal ring 23, has a substantially T shaped cross section and includes a radially oriented wall 34, which protrudes from the distal ring 23. At its free end, the wall 34 is provided with two wings 36, which extend from the wall 34 in a substantially perpendicular direction. Each projecting locking member 32 can further include a ramped nose 38, which tapers in a distal direction. In some embodiments, each projecting locking member 32 includes two parallel guiding ears 37. The guiding ears 37 protrude from the proximal ring 25 on the side of the wings 36.

The annular head 14 and the tubular body 12 are linked by the two articulated actuation arms 16, which each includes a proximal link 39 and a distal link 40.

The proximal link 39 has a substantially rectangular shape and is connected to the annular head 14 by a first proximal hinge 42. The annular head 14 has two spaced apart parallel arms 43, which are connected to two corresponding arms 44 made in the proximal link 39. The first proximal hinge 42 thus connects the two arms 43 and the two arms 44 of the proximal link 39. The two arms 44 define a window 45 in the proximal link 39. In the illustrated embodiment, the first proximal hinge 42 is a living hinge formed by a thinning of the plastic material, which the safety device 10 is made of. This allows the proximal link 39 to rotate relative to the arms 43 and 44. The proximal links 39 further may comprise two locking hooks 46, which protrude from its internal face. The two locking hooks 46 are sized and shaped to create a snap fit engagement with the wings 36 of the projecting locking member 32. To this end, the locking hooks 46 can include a ramped finger 48 and the wings 36 can also have a tapered cross section. It can be envisaged to provide the proximal link 39 with a single locking hook 46.

In some embodiments, the distal link 40 has a substantially rectangular shape and is connected to the tab 20 by a second distal hinge 47 which can be a living hinge made by a thinning of the plastic material which forms the safety device 10. As shown more clearly on Figs. 3 and Figs. 4, the distal link 40 can include a proximal and a distal finger plates 29, 30 which can be substantially orthogonally positioned and are linked by a longitudinal rib 27 which rigidly joins the two finger plates 29 and 30.

The proximal link 39 and the distal link 40 are joined by an intermediate hinge 49, which can be a living hinge made by a thinning of the plastic material which forms the safety device 10.

Thus in some embodiments, the safety device 10 comprises two articulated actuation arms 16 which connect the tubular body 12 and the annular ring 14 and comprises a unitary structure integrally molded of thermoplastic material.

In some embodiments, the safety device 10 can be a single piece component. Further, the safety device 10 can be formed by a single shot molding process. The plastic polymer used in the molding process can be a suitable polymer such as thermoplastic, which provides a shape memory to the safety device 10 configured to recover its original shape from deformation when a force has been applied.

In some embodiments, in an initial and relaxed state, (see e.g. Fig.3), after manufacturing by injection molding, the safety device 10 is configured in a state wherein the annular head 14 and the tubular body 12 are axially spaced apart while the proximal link 39 and the distal link 40 form a substantially right angle. In practice, the angle between proximal link 39 and the distal link 40 can be comprised between for example 75° and 105°. When an axial force is applied to the safety device 10, the actuation arms 16 may bend about the living hinges 42, 47 and 49 and flex radially outwardly or inwardly depending on the force direction. When the force ceases to apply on the safety device 10, it regains its initial and relaxed state. The actuation arms 16 are therefore transitionable from an initial and relaxed state where the proximal link 39 and distal link 40 form a first angle - substantially right angle - to:
(i) a collapsed state where the proximal link 39 and the distal link 40 are substantially parallel to each other or at least form an acute angle, or
(ii) an extended state where the proximal link 39 and the distal link 40 form are substantially in line with each other.

Starting with a safety device 10 as shown on Fig.3, the safety device 10 can be moved into a collapsed position as shown on Fig.6.

To do so, the tubular body 12 is pushed in a proximal direction so that the legs 21 which extend from said tubular body 12 deflects against the noses 38, this being made smooth by the ramped catch 22 deflecting against the tapered nose 38. The legs 21 are then channeled between the guiding ears 37. At the end of the push, the catch 22 locks against the free end of the proximal ring 25 achieving an appropriate retention of the tubular body 12 onto the annular ring 14 as illustrated on Fig.7. In this locked state, the actuation arms 16 are pushed in a collapsed state as can be seen on Fig.6. In some embodiments, the actuation arm 16 in its collapsed state has the proximal link 39 substantially perpendicular to the axis A whereas the proximal finger plate 29 is substantially parallel to the longitudinal axis A and the distal plate is substantially perpendicular to the longitudinal axis A.

In this collapsed configuration, the safety device is in a stable state and can be shipped and stored for later use or can be immediately engaged on a drug delivery device.

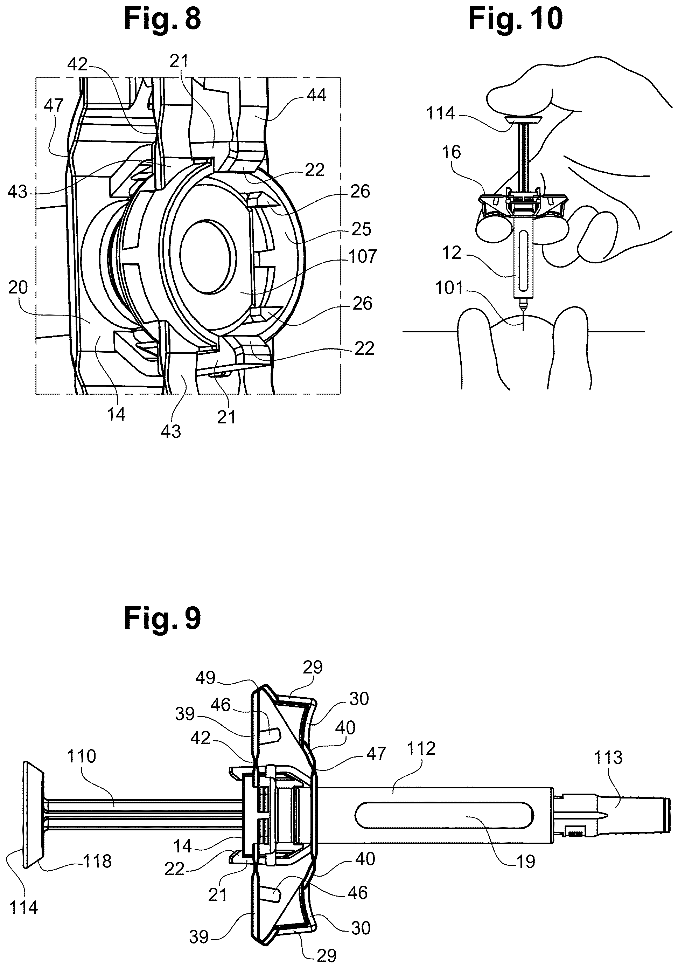

In an exemplary use, the safety device 10 can be engaged on a drug delivery device 100, which can be a prefilled syringe as illustrated on Fig.9.

The drug delivery device distal end is inserted through the annular head 14 and is pushed in a distal direction (see e.g. Fig. 9). The annular head 14 is locked onto the barrel flange 107. As best seen on Fig.8, the locking tabs 26 deflect when the annular head 14 is pushed in a proximal direction so that the annular head 14 is retained on the barrel flange 107.

The drug delivery device 100 is axially locked on the safety device 10, however the drug delivery device 100 is free to rotate relative to the safety device 10, which can be useful as user tend to rotate any drug delivery device before injection to visually check the drug which is about to be injected.

The drug delivery device fitted with a safety device 100 as illustrated on Fig.9 can be stored for a later use.

When an injection of the drug contained in the drug delivery device 100 is required, a user may first remove the tip cap 113.

The drug delivery device 100 is ready for injection. The user can visually check the drug through the windows 19 and can rotate the plunder rod 110.

Administering an injection with a drug delivery device fitted with the safety device 10 is substantially the same as administering an injection with a drug delivery device fitted with the safety device 10 according to the prior art and thus is not likely to confuse a user and permits a user-friendly operation.

In an exemplary use illustrated on Fig.10, the user can apply a pushing force on the piston rod flange 114 typically by applying a thumb on the piston rod flange 114 while the user maintains one finger - typically the index - on one distal link 40 and one finger - typically the middle finger - on the other distal link 40.

In embodiments where the distal links 40 include two orthogonal finger plates 29, 30, the distal finger plates 30 provide an intuitive and ergonomic surface to place fingers.

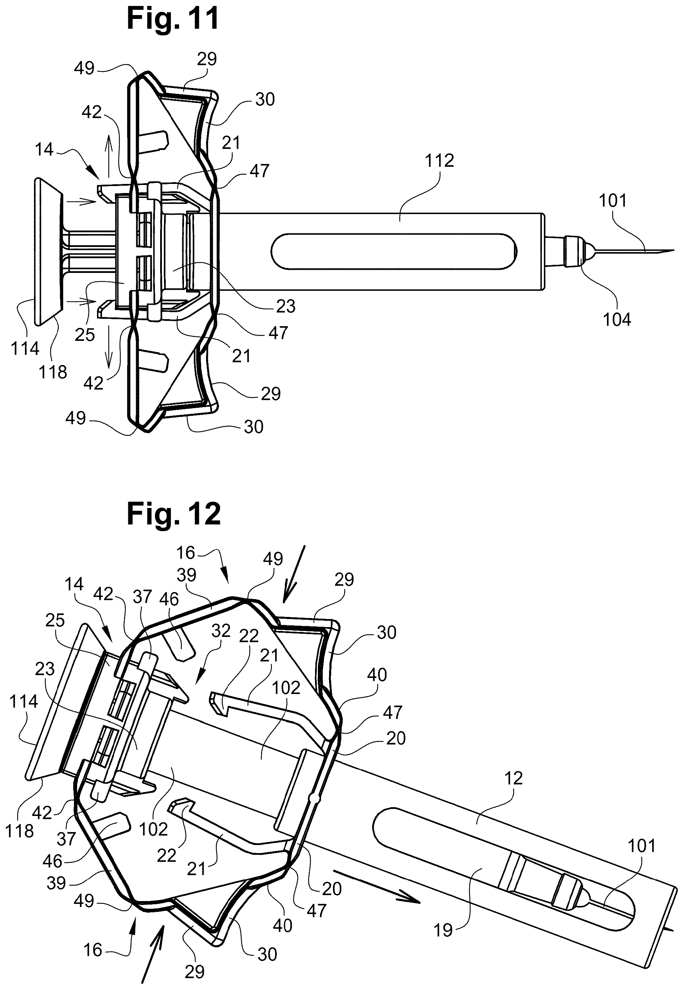

As the injection progresses, the piston rod flange 114 moves closer to the annular head 14. At the end of the injection, the piston rod flange 114 abuts against the catch 22. By further pushing the piston rod flange 114, the ramp portion 118 comes in abutment with the ramp provided in the catch 22 of each legs 21 as indicated by the arrows of Fig.11. The legs 21 are thus forced to deflect outwardly thereby releasing the catch 22. The actuation arms 16 and the tubular body 12 are released from the annular head 14 and are therefore free to move axially along the barrel 102.

In one or more embodiment, as they are free, the actuation arms 16 move toward their initial and relaxed state where the proximal link 39 and the distal link 40 form a substantially right angle depending on the material and the injection process used to manufacture the safety device 10.

As the actuations arms 16 return to their initial and relaxed position, the tubular body 12 moves axially on a distal position and covers at least partially the needle 101. However, in this state the tubular body 12 is still free to move in the proximal direction.

This is why, the safety device 10 is moved to a locked extended position. To achieve this, the user can exert a radial force on each actuation arms 16. The user can place one finger - shown by a radial arrow on Fig.12 - on the proximal finger plate 29 of one actuation arm 16 and one finger - shown by a radial arrow on Fig.12 - on the other proximal finger 29 plate of the other actuation arm 16; both proximal finger plates 29 are intuitive and friendly to use. The arrows of Fig.12 show where the user applies a force with the fingers of one hand and with the fingers of two hands.

By doing so, the actuation arms 16 move into an extended state distally pushing the tubular body 12 into a position where the tubular body 12 entirely encapsulates the needle 101.

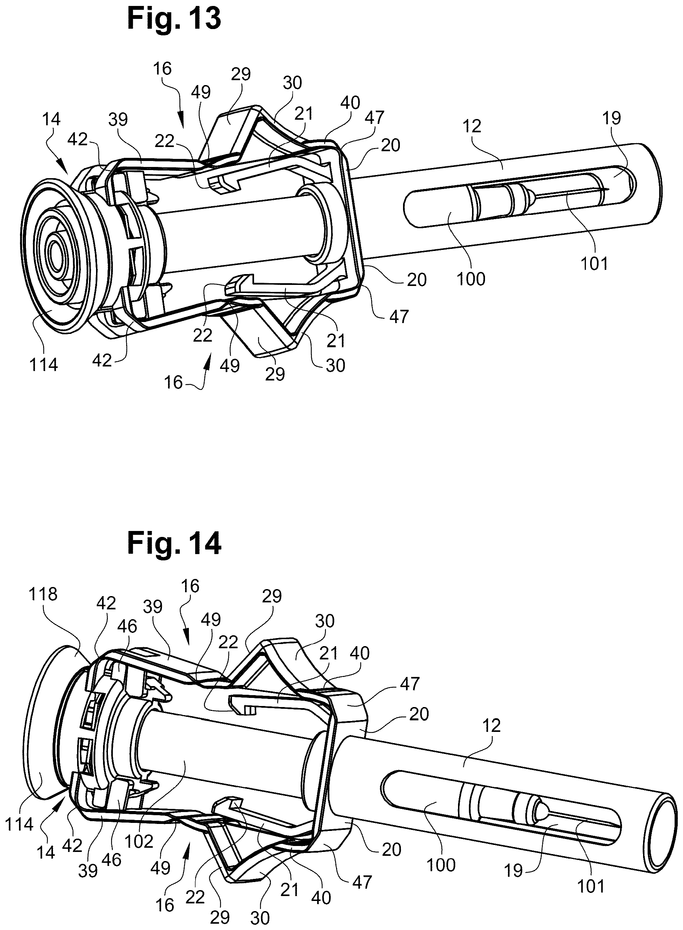

At the end of the pushing on the actuation arms 16, the two locking hooks 46 lock into the wings 36 of the projecting locking member 32 and maintain the actuation arms 16 in their respective extended positions where the tubular body 12 is pushed distally and thus the tubular body 12 shields the needle 101 from post-injection needle stick as seen in Figs 13 to 15.

In some embodiments, the engagement of the locking hooks 46 on the wings 36 produces an audible and/or a tactile signal indicating that locking is positively achieved.

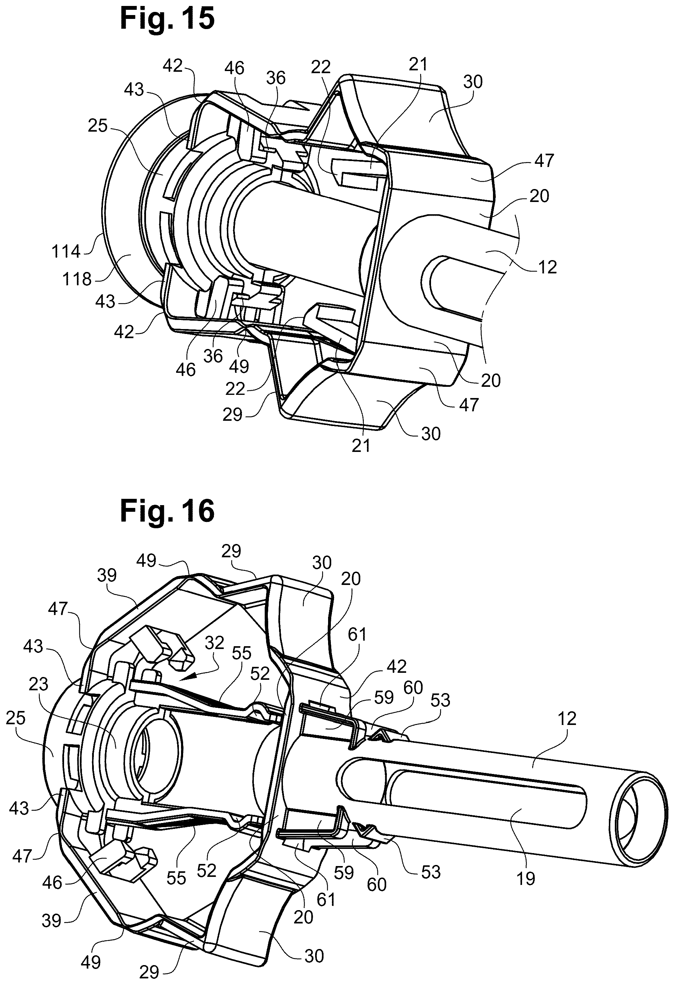

In some embodiments, the actuation arms 16 are in a state where the intermediate hinge 49 inwardly overtakes the plan defined by the proximal hinge 42 and distal hinge 47 as it can be best seen on Fig. 15. Thus, a force applied longitudinally on the tubular body 12 aiming at un-shielding the needle tip 101 cannot release the actuation arm 16 from its extended state. The tubular body 12 is locked in an extended state by the locking hooks 46; locking is further achieved by the proximal link 39 and distal link 40 which are arranged as a toggle mechanism.

The protective device 10 accordingly achieves a substantially permanent locking of the tubular body 12 over the needle tip 101.

In an embodiment of the invention illustrated on Fig 16, the annular head 12 includes two longitudinal tongues 55, which each extends distally from the locking member 32. The longitudinal tongue 55 has a substantially flat cross section and includes a corrugation 52 at its distal end.

The tubular body 12 is further provided with two parallel guiding walls 59, which extend from each tab 20 in a distal direction. Each pair of guiding walls 59 define a channel 60, configured and sized to receive the tongue 55. In the illustrated embodiment, each the tab 20 includes a window 61 to allow the tongue 55 insertion between the two guiding walls 59. At the distal end of each pair of the guiding walls 59, the tubular body includes a transverse rib 53.

While not shown, the tubular body 12 may be held in its collapsed state via one of the corrugation 52 being engaged on the transverse rib 53 while the tongue 55 is engaged between the two guiding walls 59 thereby increasing the overall rigidity of the safety device 10 when in collapsed state. The elastic snap fit of the corrugation 52 on the rib 53 can provide an audible and/or tactile feedback to a user.

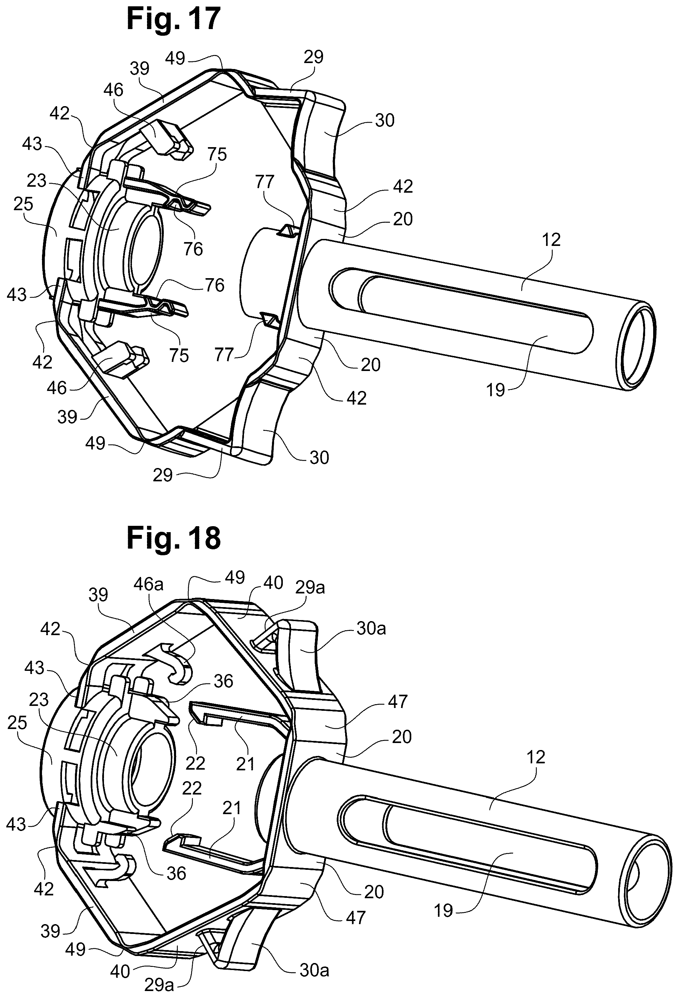

In one embodiment shown on Fig.17, the annular head 12 includes a tab 75, which extends distally from the locking member 32. The tab 75 includes a transverse corrugation 76 which is sized and configured to engage on a rib 77 provided on the annular body 12. The corrugation 76 being engaged on the rib 77 provides an axially locking force when the actuation arm 16 is in its collapsed state.

In one embodiment shown on Fig.18, the safety device 10 includes two hooks 46a, which protrude from the inner face of the two proximal link 39. The hooks 46a have a curved free end configured and sized to enter into snap fit engagement with a wing 36. Fig. 18 further shows where the finger plates protrude from each distal plates. The curved shaped hooks and the finger plates protruding from the distal link tend to make the safety device easier to manufacture by injection molding.

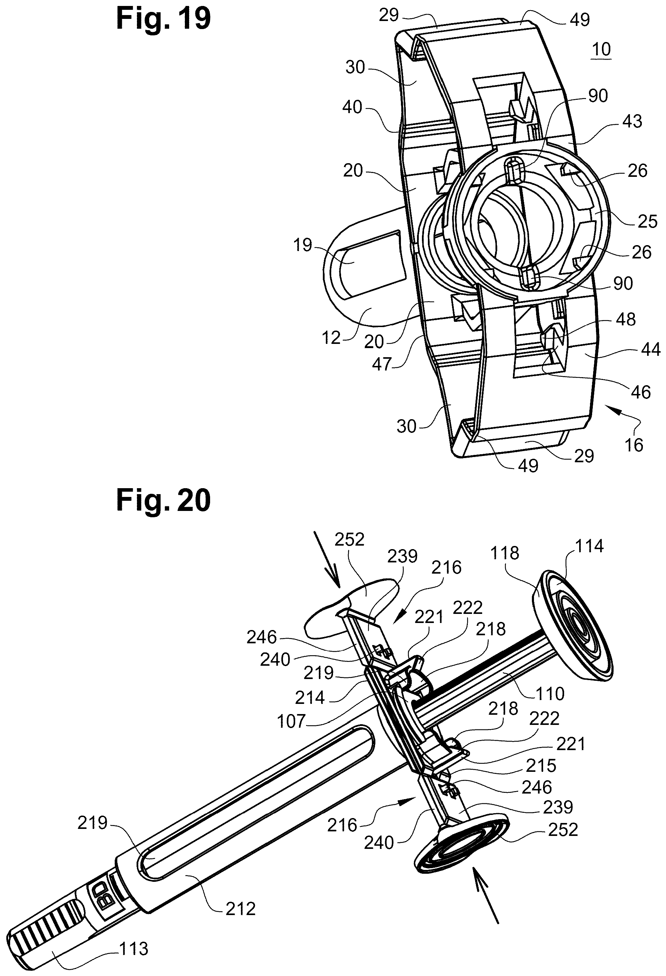

In some embodiments shown on Fig.19, the safety device 10 can include one or more recesses configured to reduce the gross weight of the safety device 10. Reducing the gross weight further means that less raw plastic injection material is needed. Manufacturing cycles can be reduced as less material is required and cooling time is improved. In the illustrated embodiment of Fig.19, two recesses 90 are provided in the annular ring 14. The recesses 90 can be positioned at the junction between the shoulder 24 and the distal ring 23. In one or more embodiments, the recesses 90 can be located above the projecting locking member 32.

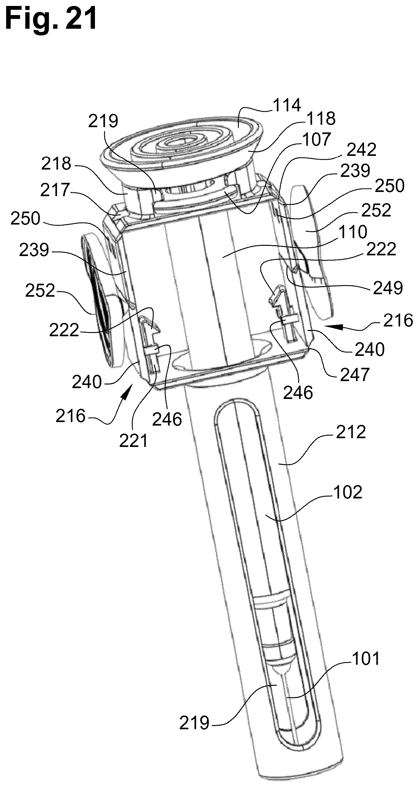

Turning to Figs.20 to Fig.24, the safety device 210 includes a tubular body 212, and is configured and sized to engage on the barrel 102 of a drug delivery device such as a prefilled syringe and a head arrangement which can include a crown 213 designed and configured to axially move between a locked collapsed position (Fig.20) and a locked extended position (Fig.21).

The tubular body 212 is a tubular element, which has an inner diameter sized and configured so that the tubular body 212 can axially slide relative to the barrel 102. In practice, the tubular body 212 inner diameter slightly exceeds the external diameter of the barrel 102. In some embodiments, the tubular body 212 can be provided with one or more windows 219 so that any marking or labelling applied on the barrel 102 and so that the drug contained in the barrel 102 can be visible to a user prior to injection. In the illustrated embodiments, the tubular body 212 is provided with two windows 219 spaced apart by 180°.

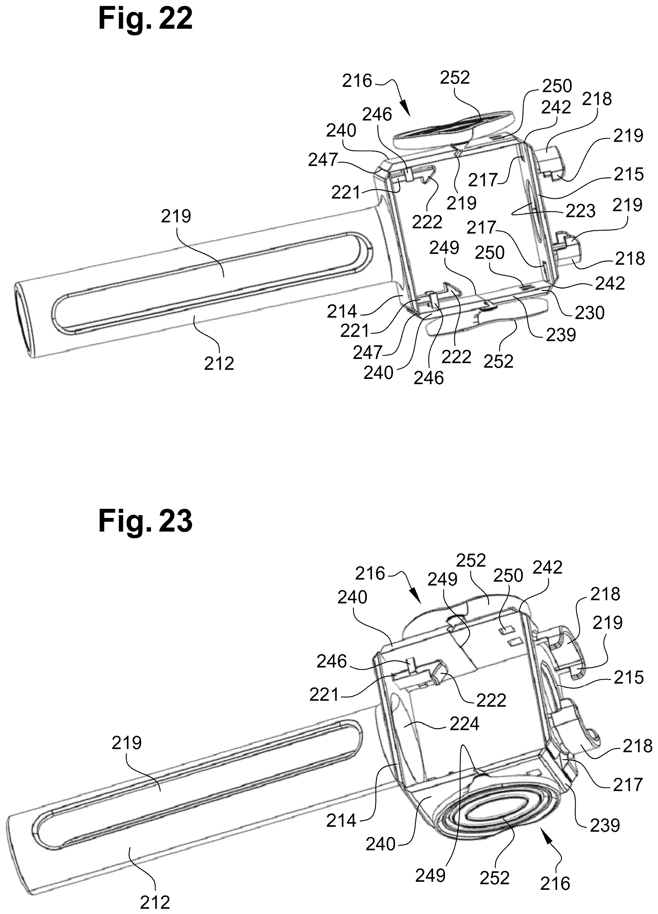

The crown 213 includes a distal plate 214 which is positioned at the proximal end of the tubular body 212. In the illustrated embodiment, the distal plate 214 has a quadrangular shape. As it can be seen on Fig.22 and Fig.23, the distal plate 214 includes two symmetrical legs 221. The legs 221 extend in cantilever the distal plate 214 in the proximal direction. The cantilevered attachment of the legs 221 onto the distal plate 214 provides the legs 21 with elastic resilience in a radial direction relative to the tubular body 212. At the end of the legs 221, is a catch 222, which is internally oriented. In some embodiments, the catch 222 has a ramped end.

The crown 213 includes a proximal plate 215 which can have a quadrangular shape. On its proximal face, the proximal plate 215 is provided with locking elements where the flange 107 can engage the locking elements.

The proximal plate 215 and the distal plate 214 are provided with two coaxial circular openings 223 and 224.

In some embodiments, the proximal plate 215 can include two symmetrical ribs 218. Each ribs 218 can be provided with two cantilevered locking tooth 219.

The proximal plate 215 can include two windows 217 which are each adjacent a ribs 218. The windows 217 are configured and sized to receive a leg 221. In other words, the legs 221 can engage the windows 217 and the catches 222 can lock onto the ribs 218 when the safety device 210 is in a collapsed position.

The crown 213 further includes two arms 216 which connect the distal plate 214 to the proximal plate 215. Each arm 216 includes a proximal link 239 and a distal link 240.

The proximal links 239 have a substantially rectangular shape and are each connected to proximal plate 216 by a first proximal hinge 242. In the illustrated embodiment, the first proximal hinges 242 are living hinges formed by a thinning of the plastic material, which the safety device 210 is made of. This allows the proximal links 239 to rotate relative to proximal plate 215. The proximal links 239 can be provided with two openings 250.

The distal links 240 have a substantially rectangular shape and are each connected to the distal plate 214 by a distal hinge 247 which can be a living hinge made by a thinning of the plastic material which forms the safety device 10.

The proximal link 239 and the distal link 240 can be joined by an intermediate hinge 249, which can be a living hinge made by a thinning of the plastic material which forms the safety device 10.

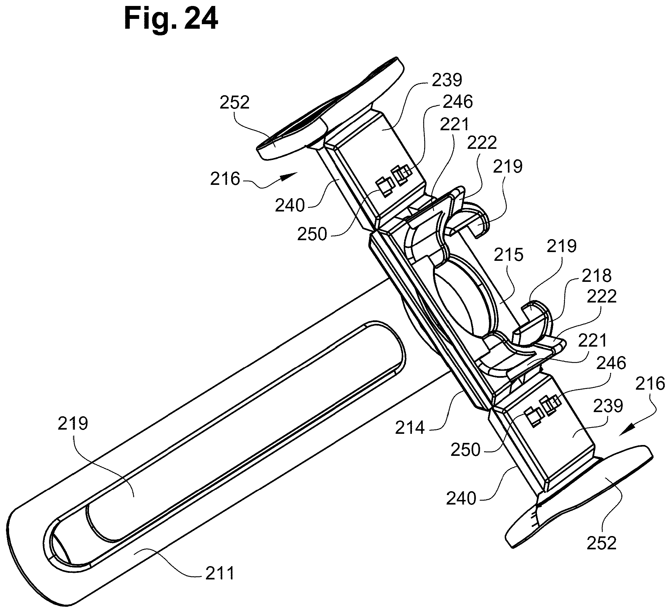

The distal links 240 can each include two locking hooks 246, which protrude from its internal face. The locking hooks 246 are sized and shaped to create (i) a snap fit engagement with the openings 250 provided in the proximal links 239 where the proximal links 239 and the distal links 240 are substantially parallel see Fig.24 and (ii) a snap fit engagement with the legs 221 where the proximal links 239 and the distal links 240 are in line with each other substantially see Fig. 22 and Fig.23.

In some embodiments, the arms 216 can be each provided with finger tabs 252. The finger tabs 252 can be positioned at the intermediate hinges 249.

The actuation arms 216 are therefore transitionable between:
(i) a collapsed state where the proximal link 239 and the distal link 240 are substantially parallel to each other, or
(ii) an extended state where the proximal link 239 and the distal link 240 form are substantially in line with each other.

In some embodiments, the safety device 210 can be a single piece component. Further, the safety device 210 can be formed by a single shot molding process.

In its collapsed position shown on Fig.24, the proximal link 239 and the distal link 240 are parallel and are maintained against each other by the locking hooks 246 which engage into the openings 250. Further, the legs 221 engage the windows 217 and lock into the ribs 218.

In its extended position shown on Figs. 23 and 24, the proximal link 239 and the distal link 240 are in line with each other and the locking hooks 246 retain the legs 221.

Fig.20 shows the safety device 210 fitted on a drug delivery device 100 which can be a prefilled syringe.

In this collapsed position, the crown 213 is locked onto the drug delivery device 100. To do so, the barrel flange 107 locks onto the ribs 108. The barrel flange 107 snaps fit onto the locking tooth 219.

The drug delivery device 100 is axially locked on the safety device 210, however the drug delivery device 100 is free to rotate relative to the safety device 10, which can be useful as user tend to rotate any drug delivery device before injection to visually check the drug which is about to be injected.

The drug delivery device fitted with a safety device 100 as illustrated on Fig.20 can be stored for a later use.

When an injection of the drug contained in the drug delivery device 100 is required, a user may first remove the tip cap 113.

The drug delivery device 100 is ready for injection. The user can visually check the drug through the windows 19 and can rotate the plunder rod 110.

Administering an injection with a drug delivery device fitted with the safety device 210 is substantially the same as administering an injection with a drug delivery device fitted with the safety device 10 according to the prior art and thus is not likely to confuse a user and permits a user-friendly operation.

In an exemplary use illustrated on Fig.10, the user can apply a pushing force on the piston rod flange 114 typically by applying a thumb on the piston rod flange 114 while the user maintains one finger - typically the index - on one distal link 240 and one finger - typically the middle finger - on the other distal link 240.

As the injection progresses, the piston rod flange 114 moves closer to the crown 214. At the end of the injection, the piston rod flange 114 abuts against the catches 222. By further pushing the piston rod flange 114, the ramp portion 118 comes in abutment with the ramp provided in the catch 222 of each legs 221 as indicated by the arrows of Fig.20. The legs 221 are thus forced to deflect outwardly thereby releasing the catches 222 from the ribs 218. The actuation arms 216 and the tubular body 212 are released from the proximal plate 215 and are therefore free to move axially along the barrel 102.

The user can exert a radial force on each actuation arms 216. To do so, the user can place one finger on one finger tab 252 and another finger on the other finger tab 252.

At the end of the pushing on the actuation arms 216, the two locking hooks 246 lock into the legs 221 and maintain the actuation arms 216 in their respective extended positions where the tubular body 212 is pushed distally and thus the tubular body 212 shields the needle 101 from post-injection needle stick as seen on Fig.21.

In some embodiments, the engagement of the locking hooks 246 on the legs 221 produces an audible and/or a tactile signal indicating that locking is positively achieved.

The terminology used herein is for the purpose of describing particular aspects only and is not intended to be limiting of the disclosure. As used herein, the singular forms "a," "an," and "the" are intended to include the plural forms as well, unless the context clearly indicates otherwise. As used herein, the term "and/or" includes any and all combinations of one or more of the associated listed items. It will be further understood that the terms "comprises," "comprising," "includes," and/or "including" when used herein specify the presence of stated features, integers, steps, operations, elements, and/or components, but do not preclude the presence or addition of one or more other features, integers, steps, operations, elements, components, and/or groups thereof.

It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure.

Relative terms such as "below" or "above" or "upper" or "lower" or "horizontal" or "vertical" may be used herein to describe a relationship of one element to another element as illustrated in the Figures. **It** will be understood that these terms and those discussed above are intended to encompass different orientations of the device in addition to the orientation depicted in the Figures. **It** will be understood that when an element is referred to as being "connected" or "coupled" to another element, it can be directly connected or coupled to the other element, or intervening elements may be present. In contrast, when an element is referred to as being "directly connected" or "directly coupled" to another element, there are no intervening elements present.

Unless otherwise defined, all terms (including technical and scientific terms) used herein have the same meaning as commonly understood by one of ordinary skill in the art to which this disclosure belongs. **It** will be further understood that terms used herein should be interpreted as having a meaning consistent with their meaning in the context of this specification and the relevant art and will not be interpreted in an idealized or overly formal sense unless expressly so defined herein.

**It** is to be understood that the present disclosure is not limited to the aspects described above and illustrated in the drawings; rather, the skilled person will recognize that changes and modifications may be made within the scope of the appended claims.

## Claims

1. A safety device (10,210) for mounting onto a drug delivery device (100) having a barrel (102) including with a flange (107) at its proximal end and an injection needle (101) at its distal end, and a piston rod (110) including a piston flange (114), the safety device (10) including:
a tubular body (12,212) extending along a longitudinal axis (A), the tubular body (12,212) being configured to receive the barrel (102),
a head arrangement (14,23,24,25) configured to abut against the flange (107) and to lock into the flange (107), the safety device being **characterized by**
at least two actuation arms (16,216) connecting the tubular body (12) and the head arrangement, said actuation arms (16,216) having a proximal link (39,239) connected to the head arrangement by a proximal hinge (42,242) and a distal link (40,240) connected to the tubular body (12,212) by a distal hinge (47,247), the proximal link (39,239) and the distal link (40,240) being connected by an intermediate hinge (49,249), the actuation arms (16,216) being movable between a collapsed position in which the tubular body (12,212) is maintained in a position proximally close to the flange (107) uncovering the needle (101) and an extended position in which the tubular body (12,212) is maintained in a position distally distant from the head arrangement shielding the needle (101);
a locking means (21 ,22,52,53,76,77,246,250) for releasably maintaining the actuation arms (16,216) in said collapsed position and a locking means (36,46,221 ,246) for maintaining the actuation arms (16,216) in said extended position.

2. The safety device (10,210) of claim 1, wherein at least one the hinge (42, 47, 49, 242, 247, 249) is a living hinge.

3. The safety device (10,210) of claim 1 or claim 2, wherein the safety device (10) is formed in a single shot injection molding process.

4. The safety device (10,210) of any of claims 1 to 3, wherein the safety device (10,210) has a relaxed state in which the arms (16,216) returns from being in the collapsed position or from being in the extended position.

5. The safety device (10) of any of claims 1 to 4, wherein the proximal link (39) and the distal link (40) form an angle between 75° and 105° in the relaxed position.

6. The safety device (10) of any of claims 1 to 5, wherein the proximal links (39) include at least one locking hooks (46) configured and sized to enter into resilient snap fit engagement with at least one wings (36) protruding from the annular head (14) to maintain the actuation arm (16) in the extended position.

7. The safety device (10) of any of claims 1 to 6, wherein the head arrangement includes an annular head (14) comprising a locking member (32) which includes a radially oriented wall (34) and two wings (36) extending laterally from the wall (34).

8. The safety device (10) of claims 7, wherein the annular head (14) includes a longitudinal tongue (55) which extends distally from the locking member (32) and is configured and sized to engage into a channel (60) defined by two guiding walls (59) provided on the tubular body (12).

9. The safety device (10) of any claims 1 or 8, wherein the intermediate hinge (49) inwardly overtakes the plan defined by the proximal hinge (42) and distal hinge (47) when the actuation arm (16) is in its extended position.

10. The safety device (10) of any claims 5 to 9 wherein the annular head (16) includes a proximal ring (25) and a distal ring (23) jointed by a shoulder (24) whereon the flange (107) abuts.

11. The safety device (10) of any of claims 1 to 10, wherein the distal link (40) includes two orthogonal finger plates (29,30).

12. The safety device (10) of any of claims 1 to 11, wherein the annular head comprises at least one locking tab (26) configured to retain the flange (107).

13. The safety device (10) of any of claims 1 to 12, wherein the tubular body (12) comprises a leg (21) extending in cantilever and having a catch (22) at its free end, the leg (21) being configured to deflect radially outward to allow the catch (22) to slide over the annular head (12) during mounting and to deflect radially inward after mounting completion so that the catch (22) axially retains the tubular body (12) relative to annular head (14).

14. The safety device (10) of any of claims 10 to 13, wherein the safety device (10) includes at least one recess (90) positioned at the junction between the shoulder (24) and the distal ring (23).

15. The safety device (210) of any of claims 1 to 3, wherein the head arrangement includes a crown (213) having a proximal plate (215) configured and sized to lock onto the flange (107) and a distal plate (214) mounted on the tubular body (212), the proximal plate (215) and the distal plate (214) being connected by at least two actuation arms (216).

16. The safety device (210) of claim 15, wherein the proximal plate (215) includes two symmetrical ribs (218), each rib (218) having two locking teeth (219) configured to retain the flange (107).

17. The safety device (210) of claim 15 or claim 16, wherein the distal link (240) includes at least one looking hook (246) configured and sized to engage into at least one opening (250) provided in the proximal link (239) when the arm (216) is in a collapsed position.

18. The safety device (210) of any of claims 15 to 17, wherein the safety device includes two legs (221) having a catch (222), extending in cantilever from the distal plate (214), configured and sized to engage into a window (217) provided in the proximal plate (215) and to lock into the rib (218).

19. The safety device (210) of any of claims 15 to 18, wherein the safety device includes at least one finger tab (252) positioned at the junction of the distal link (240) and the proximal link (239).

20. A drug delivery device (100) having a barrel (102) including a flange (107) at its proximal end and an injection needle (101) at its distal end, and a piston rod (110) including a piston flange (114) fitted with a safety device (10,210) according to one of claims 1 to 13, wherein the piston flange (114) includes a ramped surface (118) distally convergent configured to outwardly deflect the locking means maintaining the actuation arms (16,216) in a collapsed position.

## Patentansprüche

1. Sicherheitsvorrichtung (10,210) zur Montage an einer Arzneimittelabgabevorrichtung (100), die einen Zylinder (102) aufweist, beinhaltend einen Flansch (107) an seinem proximalen Ende und eine Injektionsnadel (101) an seinem distalen Ende, und eine Kolbenstange (110), beinhaltend einen Kolbenflanschs (114), wobei die Sicherheitsvorrichtung (10) Folgendes beinhaltet:
einen Rohrkörper (12,212), der sich entlang einer Längsachse (A) erstreckt, wobei der Rohrkörper (12,212) so eingerichtet ist, dass er das Rohr (102) aufnimmt,
eine Kopfanordnung (14,23,24,25), die so eingerichtet ist, dass sie an dem Flansch (107) anliegt und in dem Flansch (107) einrastet, wobei die Sicherheitsvorrichtung durch Folgendes gekennzeichnet ist
mindestens zwei Betätigungsarme (16,216), die den Rohrkörper (12) und die Kopfanordnung verbinden, wobei die Betätigungsarme (16,216) ein proximales Glied (39,239) aufweisen, das mit der Kopfanordnung durch ein proximales Scharnier (42,242) verbunden ist, und ein distales Glied (40,240), das mit dem Rohrkörper (12,212) durch ein distales Scharnier (47,247) verbunden ist, wobei das proximale Glied (39,239) und das distale Glied (40,240) durch ein Zwischenscharnier (49,249) verbunden sind, wobei die Betätigungsarme (16,216) beweglich zwischen einer eingefahrenen Position, in der der Rohrkörper (12, 212) in einer Position proximal nahe am Flansch (107) gehalten wird, der die Nadel (101) abdeckt, und einer ausgefahrenen Position, in der der Rohrkörper (12, 212) in einer Position gehalten wird, die distal von der Kopfanordnung entfernt ist, die die Nadel (101) abschirmt;
ein Verriegelungsmittel (21,22,52,53,76,77,246,250) zum lösbaren Halten der Betätigungsarme (16,216) in der eingefahrenen Position und ein Verriegelungsmittel (36,46,221,246) zum Halten der Betätigungsarme (16,216) in der ausgefahrenen Position.

2. Sicherheitsvorrichtung (10,210) nach Anspruch 1, wobei mindestens eines der Scharniere (42, 47, 49, 242, 247, 249) ein lebendes Scharnier ist.

3. Sicherheitsvorrichtung (10,210) nach Anspruch 1 oder 2, wobei die Sicherheitsvorrichtung (10) in einem Einzelspritzgussverfahren gebildet ist.

4. Sicherheitsvorrichtung (10,210) nach einem der Ansprüche 1 bis 3, wobei die Sicherheitsvorrichtung (10,210) einen entspannten Zustand aufweist, in dem die Arme (16,216) aus der eingefahrenen Position oder aus der ausgefahrenen Position zurückkehren.

5. Sicherheitsvorrichtung (10) nach einem der Ansprüche 1 bis 4, wobei das proximale Glied (39) und das distale Glied (40) in der entspannten Position einen Winkel zwischen 75° und 105° bilden.

6. Sicherheitsvorrichtung (10) nach einem der Ansprüche 1 bis 5, wobei die proximalen Glieder (39) mindestens einen Verriegelungshaken (46) beinhalten, der so eingerichtet und dimensioniert ist, dass er in eine elastische Schnappverbindung eingreift, wobei mindestens ein Flügel (36) aus dem Ringkopf (14) herausragt, um den Betätigungsarm (16) in der ausgefahrenen Position zu halten.

7. Sicherheitsvorrichtung (10) nach einem der Ansprüche 1 bis 6, wobei die Kopfanordnung einen Ringkopf (14) umfasst, der ein Verriegelungselement (32) beinhaltet, das eine radial ausgerichtete Wand (34) und zwei Flügel (36) beinhaltet, die sich seitlich von der Wand (34) erstrecken.

8. Sicherheitsvorrichtung (10) nach Anspruch 7, wobei der Ringkopf (14) eine Längszunge (55) beinhaltet, die sich distal vom Verriegelungselement (32) erstreckt und so ausgelegt und dimensioniert ist, dass sie in einen Kanal (60) eingreift, der durch zwei Führungswände (59) definiert ist, die am Rohrkörper (12) vorgesehen sind.

9. Sicherheitsvorrichtung (10) nach einem der Ansprüche 1 oder 8, wobei das Zwischenscharnier (49) die durch das proximale Scharnier (42) und das distale Scharnier (47) definierte Ebene nach innen überschreitet, wenn sich der Betätigungsarm (16) in seiner ausgefahrenen Position befindet.

10. Sicherheitsvorrichtung (10) nach einem der Ansprüche 5 bis 9, wobei der Ringkopf (16) einen proximalen Ring (25) und einen distalen Ring (23) beinhaltet, die durch eine Schulter (24) verbunden sind, auf der der Flansch (107) aufliegt.

11. Sicherheitsvorrichtung (10) nach einem der Ansprüche 1 bis 10, wobei das distale Glied (40) zwei orthogonale Fingerplatten (29, 30) beinhaltet.

12. Sicherheitsvorrichtung (10) nach einem der Ansprüche 1 bis 11, wobei der Ringkopf mindestens eine Verriegelungslasche (26) umfasst, die so eingerichtet ist, dass sie den Flansch (107) hält.

13. Sicherheitsvorrichtung (10) nach einem der Ansprüche 1 bis 12, wobei der Rohrkörper (12) einen Schenkel (21) umfasst, der sich im Ausleger erstreckt und einen Riegel (22) an seinem freien Ende aufweist, wobei der Schenkel (21) so eingerichtet ist, dass er sich radial nach außen abbiegt, um es dem Riegel (22) zu ermöglichen, während der Montage über den Ringkopf (12) zu gleiten, und sich radial nach Abschluss der Montage nach innen abbiegt, sodass der Riegel (22) den Rohrkörper (12) axial relativ zum Ringkopf (14) hält.

14. Sicherheitsvorrichtung (10) nach einem der Ansprüche 10 bis 13, wobei die Sicherheitsvorrichtung (10) mindestens eine Vertiefung (90) beinhaltet, die am Übergang zwischen der Schulter (24) und dem distalen Ring (23) positioniert ist.

15. Sicherheitsvorrichtung (210) nach einem der Ansprüche 1 bis 3, wobei die Kopfanordnung eine Krone (213) beinhaltet, die eine proximale Platte (215) aufweist, die so eingerichtet und dimensioniert ist, dass sie auf dem Flansch (107) und einer distalen Platte (214), die auf dem Rohrkörper (212) montiert ist, einrastet, wobei die proximale Platte (215) und die distale Platte (214) durch mindestens zwei Betätigungsarme (216) verbunden sind.

16. Sicherheitsvorrichtung (210) nach Anspruch 15, wobei die proximale Platte (215) zwei symmetrische Rippen (218) beinhaltet, wobei jede Rippe (218) zwei Verriegelungszähne (219) aufweist, die so eingerichtet sind, dass sie den Flansch (107) halten.

17. Sicherheitsvorrichtung (210) nach Anspruch 15 oder Anspruch 16, wobei das distale Glied (240) mindestens einen Sichthaken (246) beinhaltet, der so eingerichtet und dimensioniert ist, dass er in mindestens eine Öffnung (250) eingreift, die in dem proximalen Glied (239) vorgesehen ist, wenn sich der Arm (216) in einer eingefahrenen Position befindet.

18. Sicherheitsvorrichtung (210) nach einem der Ansprüche 15 bis 17, wobei die Sicherheitsvorrichtung zwei Schenkel (221) mit einer Sperre (222) beinhaltet, die sich im Ausleger von der distalen Platte (214) erstrecken und so ausgelegt und dimensioniert sind, dass sie in ein Fenster (217) eingreifen, das in der proximalen Platte (215) vorgesehen ist, und in der Rippe (218) einrasten.

19. Sicherheitsvorrichtung (210) nach einem der Ansprüche 15 bis 18, wobei die Sicherheitsvorrichtung mindestens eine Fingerlasche (252) beinhaltet, die am Übergang des distalen Glieds (240) und des proximalen Glieds (239) positioniert ist.

20. Arzneimittelabgabevorrichtung (100), die einen Zylinder (102) aufweist, der einen Flansch (107) an seinem proximalen Ende und eine Injektionsnadel (101) an seinem distalen Ende beinhaltet, und eine Kolbenstange (110), die einen Kolbenflansch (114) beinhaltet, der mit einer Sicherheitsvorrichtung (10,210) nach einem der Ansprüche 1 bis 13 versehen ist, wobei der Kolbenflansch (114) eine distal konvergente, abgeschrägte Oberfläche (118) beinhaltet, die so ausgelegt ist, dass sie die Verriegelungsmittel nach außen abbiegt, die die Betätigungsarme (16, 216) in einer eingefahrenen Position halten.

## Revendications

1. Dispositif de sécurité (10, 210) à monter sur un dispositif d'administration de médicament (100) ayant un cylindre (102) comprenant une bride (107) à son extrémité proximale et une aiguille d'injection (101) à son extrémité distale, et une tige de piston (110) comprenant une bride de piston (114), le dispositif de sécurité (10) comprenant :
un corps tubulaire (12, 212) s'étendant le long d'un axe longitudinal (A), le corps tubulaire (12, 212) étant configuré pour recevoir le cylindre (102),
un agencement de tête (14, 23, 24, 25) configuré pour venir en butée contre la bride (107) et se verrouiller dans la bride (107), le dispositif de sécurité étant **caractérisé par**
au moins deux bras d'actionnement (16, 216) reliant le corps tubulaire (12) et l'agencement de tête, lesdits bras d'actionnement (16, 216) ayant une liaison proximale (39, 239) reliée à l'agencement de tête par une charnière proximale (42, 242) et une liaison distale (40, 240) reliée au corps tubulaire (12, 212) par une charnière distale (47, 247), la liaison proximale (39, 239) et la liaison distale (40, 240) étant reliées par une charnière intermédiaire (49, 249), les bras d'actionnement (16, 216) étant mobiles entre une position repliée dans laquelle le corps tubulaire (12, 212) est maintenu dans une position proche de manière proximale de la bride (107) découvrant l'aiguille (101) et une position déployée dans laquelle le corps tubulaire (12, 212) est maintenu dans une position distalement éloignée de l'agencement de tête protégeant l'aiguille (101) ;
un moyen de verrouillage (21, 22, 52, 53, 76, 77, 246, 250) pour maintenir de manière amovible les bras d'actionnement (16, 216) dans ladite position repliée et un moyen de verrouillage (36, 46, 221, 246) pour maintenir les bras d'actionnement (16, 216) dans ladite position déployée.

2. Dispositif de sécurité (10, 210) de la revendication 1, dans lequel au moins une charnière (42, 47, 49, 242, 247, 249) est une charnière mobile.

3. Dispositif de sécurité (10, 210) de la revendication 1 ou 2, dans lequel le dispositif de sécurité (10) est formé par un procédé de moulage par injection en une seule fois.

4. Dispositif de sécurité (10, 210) de l'une des revendications 1 à 3, dans lequel le dispositif de sécurité (10, 210) présente un état détendu dans lequel les bras (16, 216) reviennent de la position repliée ou de la position déployée.

5. Dispositif de sécurité (10) de l'une des revendications 1 à 4, dans lequel la liaison proximale (39) et la liaison distale (40) forment un angle compris entre 75° et 105° dans la position détendue.

6. Dispositif de sécurité (10) de l'une des revendications 1 à 5, dans lequel les liaisons proximales (39) comprennent au moins un crochet de verrouillage (46) configuré et dimensionné pour entrer en prise élastique par encliquetage avec au moins une aile (36) faisant saillie depuis la tête annulaire (14) pour maintenir le bras d'actionnement (16) dans la position déployée.

7. Dispositif de sécurité (10) de l'une des revendications 1 à 6, dans lequel l'agencement de tête comprend une tête annulaire (14) comprenant un élément de verrouillage (32) qui comprend une paroi orientée radialement (34) et deux ailes (36) s'étendant latéralement depuis la paroi (34).

8. Dispositif de sécurité (10) de la revendication 7, dans lequel la tête annulaire (14) comprend une languette longitudinale (55) qui s'étend distalement depuis l'élément de verrouillage (32) et est configurée et dimensionnée pour s'engager dans un canal (60) défini par deux parois de guidage (59) prévues sur le corps tubulaire (12).

9. Dispositif de sécurité (10) de l'une des revendications 1 ou 8, dans lequel la charnière intermédiaire (49) dépasse vers l'intérieur le plan défini par la charnière proximale (42) et la charnière distale (47) lorsque le bras d'actionnement (16) se trouve dans sa position déployée.

10. Dispositif de sécurité (10) de l'une des revendications 5 à 9, dans lequel la tête annulaire (16) comprend une bague proximale (25) et une bague distale (23) jointes par un épaulement (24) sur lequel vient en butée la bride (107).

11. Dispositif de sécurité (10) de l'une des revendications 1 à 10, dans lequel la liaison distale (40) comprend deux plaques à doigts orthogonales (29, 30).

12. Dispositif de sécurité (10) de l'une des revendications 1 à 11, dans lequel la tête annulaire comprend au moins une patte de verrouillage (26) configurée pour retenir la bride (107).

13. Dispositif de sécurité (10) de l'une des revendications 1 à 12, dans lequel le corps tubulaire (12) comprend une branche (21) s'étendant en porte-à-faux et ayant un loquet (22) à son extrémité libre, la branche (21) étant configurée pour dévier radialement vers l'extérieur pour permettre au loquet (22) de glisser sur la tête annulaire (12) pendant le montage et pour dévier radialement vers l'intérieur après achèvement du montage de sorte que le loquet (22) retient axialement le corps tubulaire (12) par rapport à la tête annulaire (14).

14. Dispositif de sécurité (10) de l'une des revendications 10 à 13, dans lequel le dispositif de sécurité (10) comprend au moins un évidement (90) positionné à la jonction entre l'épaulement (24) et la bague distale (23).

15. Dispositif de sécurité (210) de l'une des revendications 1 à 3, dans lequel l'agencement de tête comprend une couronne (213) ayant une plaque proximale (215) configurée et dimensionnée pour se verrouiller sur la bride (107) et une plaque distale (214) montée sur le corps tubulaire (212), la plaque proximale (215) et la plaque distale (214) étant reliées par au moins deux bras d'actionnement (216).

16. Dispositif de sécurité (210) de la revendication 15, dans lequel la plaque proximale (215) comprend deux nervures symétriques (218), chaque nervure (218) ayant deux dents de verrouillage (219) configurées pour retenir la bride (107).

17. Dispositif de sécurité (210) de la revendication 15 ou 16, dans lequel la liaison distale (240) comprend au moins un crochet de verrouillage (246) configuré et dimensionné pour s'engager dans au moins une ouverture (250) prévue dans la liaison proximale (239) lorsque le bras (216) se trouve dans une position repliée.

18. Dispositif de sécurité (210) de l'une des revendications 15 à 17, dans lequel le dispositif de sécurité comprend deux branches (221) ayant un loquet (222), s'étendant en porte-à-faux depuis la plaque distale (214), configurées et dimensionnées pour s'engager dans une fenêtre (217) prévue dans la plaque proximale (215) et se verrouiller dans la nervure (218).

19. Dispositif de sécurité (210) de l'une des revendications 15 à 18, dans lequel le dispositif de sécurité comprend au moins une patte pour les doigts (252) positionnée à la jonction de la liaison distale (240) et de la liaison proximale (239).

20. Dispositif d'administration de médicament (100) ayant un cylindre (102) comprenant une bride (107) à son extrémité proximale et une aiguille d'injection (101) à son extrémité distale, et une tige de piston (110) comprenant une bride de piston (114) équipée d'un dispositif de sécurité (10, 210) selon l'une des revendications 1 à 13, dans lequel la bride de piston (114) comprend une surface en rampe (118) distalement convergente configurée pour faire dévier vers l'extérieur le moyen de verrouillage maintenant les bras d'actionnement (16, 216) dans une position repliée.
